Europäisches Patentamt

**European Patent Office**

**Office européen des brevets**

(11) Veröffentlichungsnummer: **0 063 342**
**B1**

(12) **EUROPÄISCHE PATENTSCHRIFT**

(45) Veröffentlichungstag der Patentschrift:
**21.11.84**

(51) Int. Cl.³: **A 61 M 5/30**

(21) Anmeldenummer: **82103080.6**

(22) Anmeldetag: **09.04.82**

(54) **Nadelloses Injektionsgerät.**

(30) Priorität: **16.04.81 DE 3115376**

(43) Veröffentlichungstag der Anmeldung:
**27.10.82 Patentblatt 82/43**

(45) Bekanntmachung des Hinweises auf die Patenterteilung:
**21.11.84 Patentblatt 84/47**

(84) Benannte Vertragsstaaten:
**CH DE FR GB LI**

(56) Entgegenhaltungen:
**DE - B - 1 213 958**
**US - A - 2 313 631**

(73) Patentinhaber: **HOECHST AKTIENGESELLSCHAFT,**
**Postfach 80 03 20, D-6230 Frankfurt am Main 80 (DE)**

(72) Erfinder: **Dettbarn, Hans-Jürgen, Rehbocks Ecke 4,**
**D-3550 Marburg/Lahn (DE)**
Erfinder: **Zimmermann, Josef, Auf der Krautweide 11,**
**D-6231 Sulzbach (DE)**

## Beschreibung

Die Erfindung betrifft ein nadelloses Injektionsgerät mit einer Kolbenpumpe für das zu injizierende Medium, die mit einem Antriebsmotor verbunden ist, dessen Arbeitskolben, der sich auf einer Arbeitsfeder abstützt, verschiebbar in einer zylindrischen Bohrung des Motorgehäuses angeordnet ist, in der eine Druckkammer zur Aufnahme eines Druckmittels zum Spannen der Arbeitsfeder ausgebildet ist, wobei in der Zuführung für das Druckmittel ein Einlaßventil und in der Abführung ein Auslaßventil angeordnet ist.

Injektionsgeräte der genannten Art sind aus der deutschen Auslegeschrift 1 213 958 bekannt. Die Feder des Antriebsmotors wird hydraulisch gespannt. Die Hydraulikkammer des Antriebsmotors ist im By-pass zu einem Hydraulikflüssigkeitskreislauf geschaltet, in dem ein Ausweichventil angeordnet ist. Zum Spannen der Feder muß dieses durch Bedienung eines Drückers geschlossen werden, damit die Hydraulikflüssigkeit in die Hydraulikkammer gelangen kann. Für die Druckhaltung ist ein Kugelkontrollventil in der By-pass-Leitung erforderlich. Zum Entspannen der Arbeitsfeder muß durch Bedienen eines zweiten Drückers ein zweites Kontrollventil offengehalten werden, wodurch für die in der Hydraulikkammer unter Druck stehende Flüssigkeit der Weg zurück in den Vorratsbehälter für die Hydraulikflüssigkeit freigegeben wird. Nachteilig ist, daß beide Drücker gleichzeitig betätigt werden können. Das Füllen der Hydraulikkammer ist somit nicht zwangsläufig gewährleistet, wodurch Fehldosierungen für das zu injizierende Medium nicht auszuschließen sind.

Hier will die Erfindung Abhilfe schaffen. Die Erfindung, wie sie in den Ansprüchen gekennzeichnet ist, löst die Aufgabe dadurch, daß den Ventilen eine Steuerwelle zugeordnet ist, die mit einem Hebel fest verbunden ist, wobei die Steuerwelle segmentartige Ausnehmungen aufweist, die den Eingriff der Ventile mit der Steuerwelle unterbrechen und so in bezug auf die Ventile angeordnet sind, daß die Ventile sich nicht gleichzeitig im Eingriff mit der Steuerwelle befinden.

Dem Hebel können Stößel für seine Bedienung zugeordnet sein, die mit einem Bedienungselement in Verbindung stehen.

Die durch die Erfindung erreichten Vorteile sind im wesentlichen darin zu sehen, daß durch die den Ventilen gemeinsame Steuerwelle Fehlbedienungen beim Zuführen und Abführen des Druckmittels in die bzw. aus der Druckkammer des Arbeitsmotors vermieden werden.

Im folgenden wird die Erfindung anhand von lediglich einen Ausführungsweg darstellenden Zeichnungen näher erläutert. Es zeigt

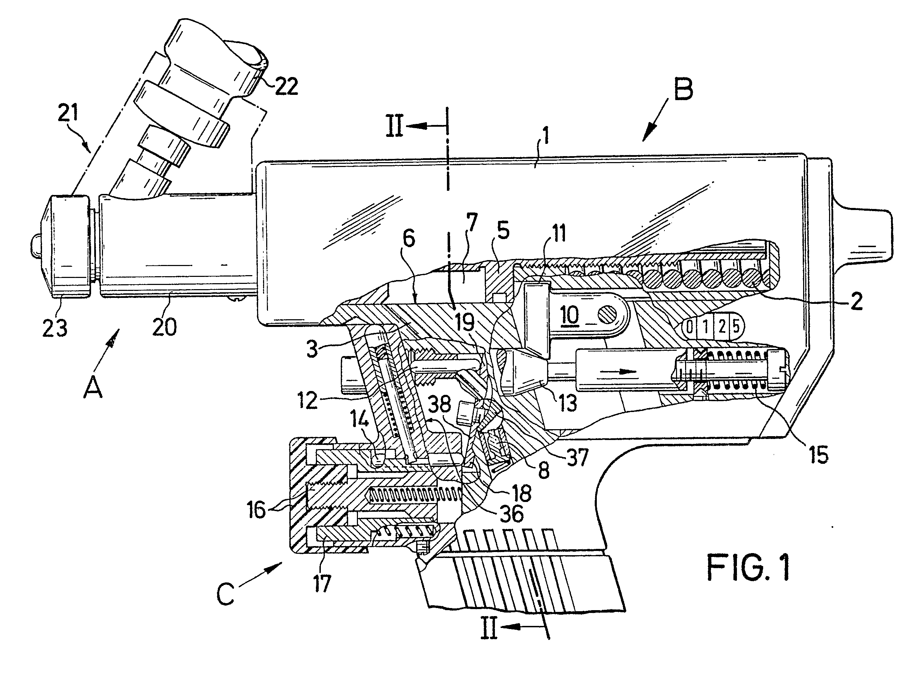

Fig. 1 eine Seitenansicht des Injektionsgerätes teilweise und in verschiedenen Ebenen geschnitten in gespanntem Zustand.

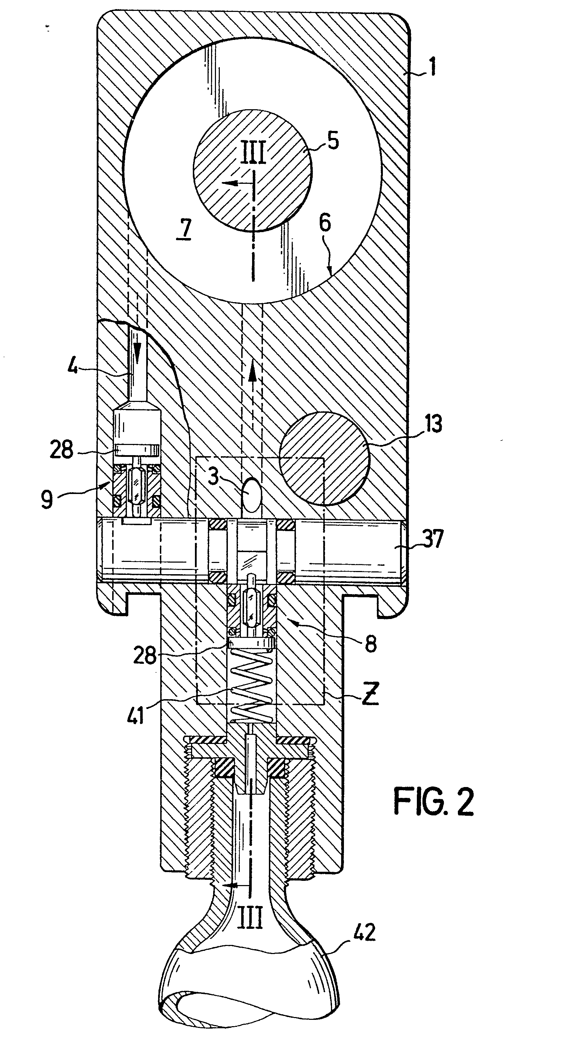

Fig. 2 den Schnitt II-II der Fig. 1.

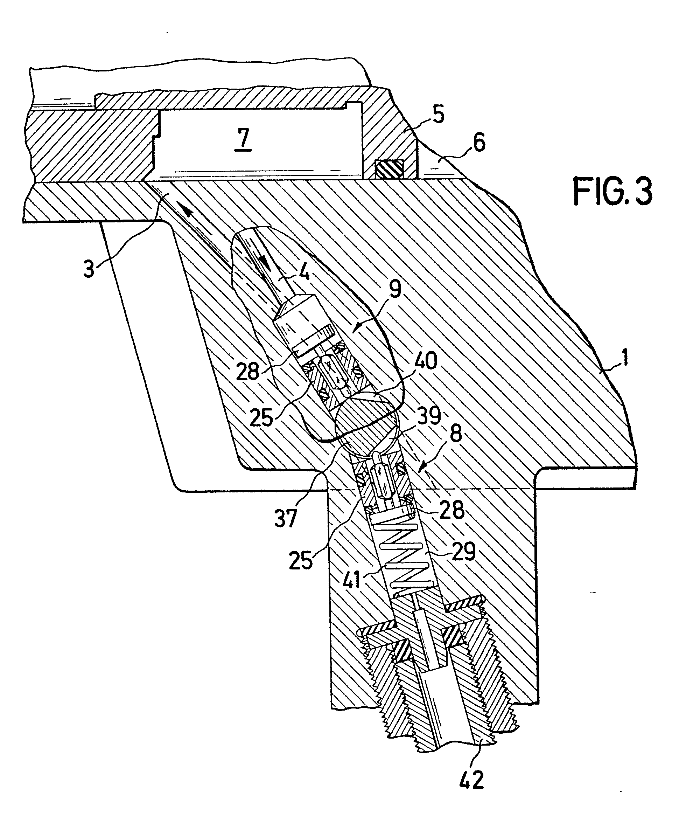

Fig. 3 den Schnitt III-III der Fig. 2.

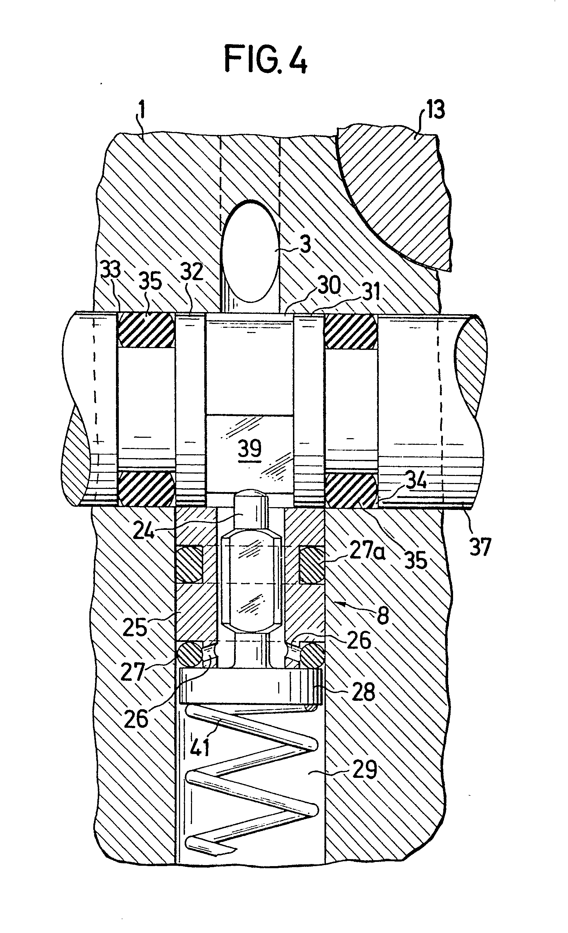

Fig. 4 die Einzelheit »Z« der Fig. 2.

Das nadellose Injektionsgerät besteht im wesentlichen aus einer Kolbenpumpe (A) für das zu injizierende Medium, die mit einem Antriebsmotor (B) verbunden ist. Das Gehäuse (20) der Kolbenpumpe (A) trägt eine Einrichtung (21) zur Aufnahme eines Behälters (22) für das zu injizierende Medium. Der Arbeitskolben (5) des Antriebsmotors (B) ist in einer zylindrischen Bohrung (6) des Motorgehäuses (1) verschiebbar angeordnet. Die Arbeitsfeder (2) des Arbeitskolbens (5) wird mit einem Druckmittel, z. B. Hydraulikflüssigkeit oder Gas gespannt, das über Einlaßventil (8) und über Kanal (3) der Druckkammer (7) in der zylindrischen Bohrung (6) zugeführt wird. Über Kanal (4) und Auslaßventil (9) wird das Druckmittel aus der Druckkammer (7) abgeführt. Einlaßventil (8) und Auslaßventil (9) ist eine Steuerwelle (37) zugeordnet, die mit einem Hebel (38) verbunden ist. Die Steuerwelle (37) weist segmentartige Ausnehmungen (39) und (40) auf, die den Eingriff der Ventile (8) und (9) mit der Steuerwelle (37) unterbrechen. Die Ausnehmungen (39) und (40) sind so auf der Steuerwelle (37) angeordnet, daß die Ventile (8) und (9) nicht gleichzeitig mit der Steuerwelle (37) in Eingriff stehen können. Zum Betätigen des Hebels (38) können diesem Stößel (12) und (14) zugeordnet sein, die mit dem Bedienungselement (C) in Verbindung stehen.

Das Bedienungselement (C) enthält unter anderem einen Bedienungsknopf (16) und eine Hülse (17). Zum Spannen des Gerätes wird der Bedienungsknopf (16) gedrückt, wodurch die Hülse (17) auf einen Stößel (14) drückt, der wiederum auf das untere Ende (18) eines mit der Steuerwelle (37) fest verbundenen Hebels (38) drückt und dadurch eine Schwenkbewegung der Steuerwelle (37) bewirkt. Die Steuerwelle (37) öffnet und schließt jeweils das Einlaßventil (8) oder das Auslaßventil (9). Das Einlaßventil (8) und das Auslaßventil (9) bestehen aus je einem Ventilteller (28) mit Führungszapfen (24) und einer Buchse (25) mit Dichtungen (27, 27a). Die Buchse (25) ist in einer Bohrung (29) im Motorgehäuse (1) angeordnet. Die Steuerwelle (37) weist im Bereich der segmentartigen Ausnehmung (39) eine Eindrehung (30) als Verteilerkanal für das Druckmittel sowie rechts und links, durch zwei schmale Schultern (31) und (32) getrennt, zwei weitere Eindrehungen (33) und (34) zur Aufnahme von Dichtungen (35) auf. Die Buchse (25) stützt sich auf den Schultern (31) und (32) ab. Bei geöffnetem Ventil (8) liegt der Führungszapfen (24) auf der zylindrischen Eindrehung (30) der Steuerwelle (37) auf. Bei geschlossenem Ventil (8) liegt der Ventilteller (28) auf der Dichtung (27) auf und der Führungszapfen (24) berührt die Steuerwelle (37) wegen der Ausnehmung (39) nicht. Die Bohrungen (26) sind zwecks Druckausgleich vorgesehen, damit Dichtung (27) beim Abheben des Ventiltellers (28) ihre Lage nicht ändert. Auslaßventil (9) ist entsprechend konzipiert.

Durch Drücken auf das Ende (18) des Hebels (38) schwenkt die Steuerwelle (37), das Einlaßventil (8) öffnet, und das Auslaßventil (9) schließt. Durch das Einströmen des Druckmittels über den Kanal (3) in die Druckkammer (7) wird die Arbeitsfeder (2) über den Arbeitskolben (5) gespannt. Sobald die Klinke (10) in die Ausnehmung (11) einrastet, wird der Riegel (13) mit dem daran befestigten Bedienungselement (C) durch die Kraft der Feder (15) in Richtung Pfeil gezogen. Dabei drückt die Fläche (36) des Bedienungselementes (C) den Stößel (12) gegen das obere Ende (19) des Hebels (38), wobei dieser die Steuerwelle (37) wieder in die Ausgangslage zurück schwenkt. Dadurch kann das Einlaßventil (8) durch Feder (41) gehalten und geschlossen werden und das Auslaßventil (9) wird geöffnet, so daß das Druckmittel aus der Druckkammer (7) über den Kanal (4) entweichen kann. Der Injektionsschuß wird durch Aufdrücken der Düse (23) auf das zu injizierende Objekt ausgelöst. (42) deutet eine Druckgasflasche an.

**Patentansprüche**

1. Nadelloses Injektionsgerät mit einer Kolbenpumpe (A) für das zu injizierende Medium, die mit einem Antriebsmotor (B) verbunden ist, dessen Arbeitskolben (5), der sich auf einer Arbeitsfeder (2) abstützt, verschiebbar in einer zylindrischen Bohrung (6) des Motorgehäuses (1) angeordnet ist, in der eine Druckkammer (7) zur Aufnahme eines Druckmittels zum Spannen der Arbeitsfeder (2) ausgebildet ist, wobei in der Zuführung (3) für das Druckmittel ein Einlaßventil (8) und in der Abführung (4) ein Auslaßventil (9) angeordnet ist, dadurch gekennzeichnet, daß den Ventilen (8, 9) eine Steuerwelle (37) zugeordnet ist, die mit einem Hebel (38) fest verbunden ist, wobei die Steuerwelle (37) segmentartige Ausnehmungen (39, 40) aufweist, die den Eingriff der Ventile (8, 9) mit der Steuerwelle (37) unterbrechen und so in bezug auf die Ventile (8, 9) angeordnet sind, daß die Ventile sich nicht gleichzeitig im Eingriff mit der Steuerwelle (37) befinden.

2. Nadelloses Injektionsgerät nach Anspruch 1, dadurch gekennzeichnet, daß dem Hebel (38) Stößel (12, 14) zugeordnet sind, die mit einem Bedienungselement (C) in Verbindung stehen.

**Claims**

1. A needle-less injection instrument with a piston pump (A), for the medium to be injected, which is connected to a drive motor (B), of which the working piston (5), supported on a working spring (2), is arranged displaceably in a cylindrical bore (6), of the motor housing (1), a pressure chamber (7) for receiving a pressure medium to tension the working spring (2), being formed in the cylindrical bore (6), and an inlet valve (8) being located in the supply line (3) for the pressure medium and an outlet valve (9) being located in the discharge line (4), wherein a control shaft (37) fixed with a lever (38) is assigned to the valves (8, 9), the control shaft (37) having segment-like recesses (39, 40) which interrupt the engagement of the valves (8, 9) with the control shaft (37) and are so arranged relative to the valves (8, 9), that the valves are not engaged simultaneously with the control shaft (37).

2. The needle-less injection instrument as claimed in claim 1, wherein rams (12, 14) connected to an operating element (C) are assigned to the lever (38).

**Revendications**

1. Appareil d'injection sans aiguille comprenant une pompe à piston (A) pour le milieu à injecter, qui est reliée à un moteur d'entraînement (B) dont le piston travaillant (5), qui s'appuie sur un ressort travaillant (2), est monté coulissant dans un alésage cylindrique (6) du corps (1) du moteur, dans lequel est formée une chambre de pression (7) destinée à recevoir un fluide sous pression servant à armer le ressort travaillant (2), une soupape d'entrée (8) étant angecée dans l'arrivée (3) du fluide sous pression et une soupape de sortie (9) étant agencée dans l'évacuation (4), caractérisé en ce qu'aux soupapes (8, 9) est associé un arbre de commande (37) qui est relié solidairement à un levier (38), l'arbre de commande (37) présentant des évidements (39, 40) en forme de segments qui interrompent la prise entre les soupapes (8, 9) et l'arbre de commande (37) et sont agencés, relativement aux soupapes (8, 9), de manière que les soupapes ne se trouvent pas simultanément en prise avec l'arbre de commande (37).

2. Appareil d'injection sans aiguille selon la revendication 1, caractérisé en ce qu'au levier (38) sont associés des poussoirs (12, 14) qui sont en liaison avec un élément de manoeuvre (C).

FIG. 1

0 063 342

0 063 342

FIG. 2

7

FIG. 3

0 063 342

# FIG. 4